# EUROPEAN PATENT APPLICATION

(11) **EP 2 597 466 A1**
(43) Date of publication of application: **29.05.2013**
(21) Application number: 12173644.1
(22) Date of filing: 26.06.2012
(51) Int. Cl.: G01N 33/68

(54) **Means and methods for proSP-B based diagnosis of alveolar damage in pulmonary hypertension patients**

(71) Applicant: Roche Diagnostics GmbH, 68305 Mannheim (DE); F. Hoffmann-La Roche AG, 4070 Basel (CH)
(72) Inventor: The designation of the inventor has not yet been filed
(74) Representative: Dick, Alexander

(57) **Abstract**

The present invention relates to the field of diagnostics. In particular, it relates to a method for diagnosing alveolar damage in a subject suffering from pulmonary hypertension, or not, said method comprising determining the amount of a surfactant protein-B peptide (SP-B peptide) in a sample of said subject, and comparing said amount to a reference amount whereby it is diagnosed whether the said subject has alveolar damage, or not. Further contemplated is a device or kit for carrying out the method of the invention.

## Description

The present invention relates to the field of diagnostics. In particular, it relates to a method for diagnosing alveolar damage in a subject suffering from pulmonary hypertension, or not, said method comprising determining the amount of a surfactant protein-B peptide (SP-B peptide) in a sample of said subject, and comparing said amount to a reference amount whereby it is diagnosed whether the said subject has alveolar damage, or not. Further contemplated is a device or kit for carrying out the method of the invention.

Pulmonary arterial hypertension (PAH) is a complex disease which is characterized by proliferation and hypertrophy of pulmonary vascular endothelial smooth muscle cells, predominantly on the arterial side of the pulmonary circulation. These abnormalities of vascular cell growth lead to intimal and medial thickening of the smaller pulmonary resistance arteries and arterioles, In some vessels these changes are so severe that they result in near obliteration of the vascular lumen and this increase resistance to blood flow though the lungs (Schermuly 2011, Nature Reviews 8: 443-455). As a consequence of PAH increased right heart filling pressures lead to right heart ventricular hypertrophy, consecutive dilatation which can be associated with reduced right and left ventricular output. The diagnosis of PAH is defined as an increased mean pulmonary artery pressure above 25 mmHg with a normal pulmonary venous pressure below 15 mmHg which requires invasive techniques (Badesch 2009, J Am Coll Cardiol 54 (Suppl) S 55 - S 66). For routine use PAH is however diagnosed by echocardiography which is by far less reliable. PAH is heterogenous (primary) PAH which has been associated with mutations of the bone morphogenic protein receptor II (BMPR II). (Mathew 2001, Cardiovascular & Hematological Agents in Medicinal Chemistry 165 - 182). Other causes include PAH due to lung disorders and hypoxia, chronic thromboembolic events, mulitfactorial mechanisms and left heart disease (Simonneau 2009, J Am Coll Cardiol 54 Siuppl: S 43 - 54). Another classification is the discrimination between pre-capillary PAH which is associated with inherited PAH and secondary pulmonary forms of PAH) and post-capillary PAH which is associated with left heart disease (Eggers 2011, Clinical Chemica Acta 412: 1582 - 1588). Severity of PAH has been associated with increased concentrations of GDF 15 (Nickel 2008, Am J. Respir Crit Care Med 178: 534 - 541), sensitive troponin T and I (Eggers 2011, Clinica Chemica Acta 412: 1582 - 1588) and BNP/NT-pro BNP (Han 2007, Circulation 116: 2992 - 3005). The disadvantage of these tests is however that they are elevated both in right and left heart disease and can not contribute to the discrimination between pre-capillary and post-capillary PAH although this information is of importance as post-capillary PAH requires treatment of left ventricular heart disease in addition to conventional treatment of PAH (Fukumoto 2011, Circulation Journal 75: 1801-1810; Mathew 2011, Cardiovascular & Hematological Agents in Medicinal Chemistry 9: 165-182).

Surfactant Proteins and, in particular, SP-B have been reported to be biomarkers for lung diseases or disorders. EP 1 845 380 A and EP 1 882 945 A disclose that mature SP-B in combination with other biomarkers are elevated in lung diseases and cardiac disorders. W01999/133337 discloses that SP-B can be used for the diagnosis of the extent of lung damage. W02004/077056 discloses that SP-B levels can be used to assess heart failure. Guazzi et al. (Guazzi 2005, Therapy 2(4): 641-648) discloses that elevated SP-B levels can, in general, be associated with alveolar-capillary membrane damage in patients with pulmonary cardiogenic edema.

The technical problem underlying the present invention can be seen as the provision of means and methods for reliably and efficiently diagnosing alveolar damage in subjects suffering from pulmonary hypertension. The technical problem is solved by the embodiments characterized in the claims and herein below.

The present invention, therefore, relates to a method for diagnosing alveolar damage in a subject suffering from pulmonary hypertension, or not, said method comprising:
a) determining the amount of a surfactant protein-B peptide (SP-B peptide) in a sample of said subject; and
b) comparing said amount to a reference amount whereby it is diagnosed whether the said subject has alveolar damage, or not.

The method of the present invention, preferably, is an in vitro method. Moreover, it may comprise steps in addition to those explicitly mentioned above. For example, further steps may relate to sample pre-treatments or evaluation of the results obtained by the method. The method may be carried out manually or assisted by automation. Preferably, step (a) and/or (b) may in total or in part be assisted by automation, e.g., by a suitable robotic and sensory equipment for the determination in step (a) or a computer-implemented comparison and/or diagnosis based on said comparison in step (b).

The method of the present invention may also, preferably, include a step of establishing a diagnosis or an aid therefor whether or not a subject suffers from a pulmonary complication, or not, based on the result of the comparison described above.

The term "diagnosing" as used herein means assessing whether a subject as referred to herein has alveolar damage, or not. As will be understood by those skilled in the art, such an assessment is usually not intended to be correct for 100% of the subjects to be diagnosed. The term, however, requires that assessment of the presence or absence of alveolar damage is correct for a statistically significant portion of the subjects (e.g. a cohort in a cohort study). Whether a portion is statistically significant can be determined without further ado by the person skilled in the art using various well known statistic evaluation tools, e.g., determination of confidence intervals, p-value determination, Student's t-test, Mann-Whitney test etc.. Details are found in Dowdy and Wearden, Statistics for Research, John Wiley & Sons, New York 1983. Preferred confidence intervals are at least 90%, at least 95%, at least 97%, at least 98% or at least 99 %. The p-values are, preferably, 0.1, 0.05, 0.01, 0.005, or 0.0001.

The term "pulmonary hypertension" as used herein refers to an increased blood pressure primarily in the pulmonary artery system. Accordingly, it is also referred to as Pulmonary arterial hypertension (PAH). It is a complex disease which is characterized by proliferation and hypertrophy of pulmonary vascular endothelial smooth muscle cells, predominantly on the arterial side of the pulmonary circulation. These abnormalities of vascular cell growth lead to intimal and medial thickening of the smaller pulmonary resistance arteries and arterioles, In some vessels these changes are so severe that they result in near obliteration of the vascular lumen and this increase resistance to blood flow though the lungs. As a consequence of PAH increased right heart filling pressures lead to right heart ventricular hypertrophy, consecutive dilatation which can be associated with reduced right and left ventricular output. The diagnosis of PAH, preferably, is defined as an increased mean pulmonary artery pressure above 25 mmHg with a normal pulmonary venous pressure below 15 mmHg which requires invasive techniques (Badesch loc. cit.). Pre-capillary hypertension can be distinguished from post-capillary hypertension based on the pulmonary capillary wedge pressure by dissecting according to a pre-capillary pressure of below about 15 mmHg and a post-capillary pressure of above about 15mmHg. For routine use, PAH is, however, preferably diagnosed by echocardiography which is by far less reliable. PAH is heterogenous (primary) PAH which has been associated with mutations of the bone morphogenic protein receptor II (BMPR II). (Mathew loc. cit.) Other causes include PAH due to lung disorders and hypoxia, chronic thromboembolic events, mulitfactorial mechanisms and left heart disease (Simonneau loc cit.). Another classification is the discrimination between pre-capillary PAH which is associated with inherited PAH and secondary pulmonary forms of PAH) and post-capillary PAH which is associated with left hear disease (Eggers loc. cit.). Severity of PAH has been associated with increased concentrations of GDF 15 (Nickel loc. cit.), sensitive troponin T and I (Eggers loc. cit.) and BNP/NT-pro BNP (Han loc. cit.). Preferably, the pulmonary hypertension as referred to in accordance with the present invention is clinically stable, more preferably, for at least several months and , preferably, at least 2, 3, 4, 5 or 6 months.

The term "alveolar damage" refers to a damage or an impairment of the alveo-capillary membrane in the lung which results in an impaired pulmonary hemodynamic. Preferably, the alveolar damage is damage of the alveolar type 2 cells, such as necrosis or apoptosis caused by alveolar hypoxia.

The term "subject" as used herein relates to animals, preferably mammals, and, more preferably, humans. The subject according to the present invention shall exhibit pulmonary hypertension as referred to elsewhere herein. Preferably, said subject does, however, not exhibit one or more other diseases or disorders selected from the group consisting of: acute cardiovascular events selected from acute cardiovascular syndrome (ACS) or myocardial infarction (MI), e.g., ST elevation MI or non-ST elevation MI, impaired kidney function (preferably, indicated by impaired creatinine clearance), other lung diseases, preferably asthma bronchiale or chronic obstructive pulmonary disease (COPD), pneumonia, lung cancer, and kidney disease. Further, the subject shall, preferably, not have been exposed to toxic gases and/or shall not be a smoker. Moreover, the subject shall, preferably, not exhibit chest pain associated with an acute cardiovascular event within at least about 2 weeks prior to sampling.

It is known that several diseases, disorders or life style behaviours, such as mentioned above, can also cause an elevation of SP-B peptides in the blood. Accordingly, the method of the present invention shall, preferably, not be applied to those subj ects. If the clinical history of a subject to be investigated by the method of the present invention with respect to the aforementioned diseases, disorders or life style behaviours is unknown, the subject may, in a preferred embodiment of the method of the present invention, be tested for the presence of the said disease, disorders and/or life style behaviours as set forth elsewhere herein.

The term "sample" refers to a sample of a body fluid. Samples of body fluids can be obtained by well known techniques and include, preferably, samples of blood, plasma, serum, or urine, more preferably, samples of blood, plasma or serum.

The term "SP-B peptide" relates to peptides which are derived from the precursor polypeptide of the Surfactant Protein B (SP-B). SP-B is synthesised as a precursor, i.e. proSP-B, and the mature SP-B is obtained by proteolytic cleavage. The mature SP-B is a 79-amino acid hydrophobic peptide that facilitates the stability and rapid spreading of surfactant phospholipids during respiratory cycles. It maintains the molecular continuity of the monolayer of the lipid and peptide at the air-water interface during breathing and facilitates the incorporation of lipids from the lung aqueous subphase into the lipid monolayer at the alveolar air-water interface. Since mature SP-B is comprised in the proSP-B in its N-terminal portion, proSP-B is, preferably, determined by detecting the presence or absence of the C-terminal portion of the proSP-B polypeptide, i.e. C-terminal proSP-B. A preferred "SP-B" in the sense of the present invention is proSP-B, preferably C-terminal proSP-B. C-terminal proSP-B in the sense of the present invention relates to proSP-B and all cleavage products or fragments thereof comprising the C-terminal sequence of full length proSP-B. Accordingly, C-terminal proSP-B includes but is not limited to the following: (i) full length proSP-B, i.e. proSP-B comprising the N-terminal propeptide including the amino acid sequence of mature SP-B and the C-terminal proSP-B, (ii) the mid-molecular portion and the C-terminal fragment, i.e. the amino acids from position 201 to 381 of proSP-B and (iii) the C-terminal proSP-B fragment, i.e. the amino acids from position 280 to 381 of proSP-B. Mature SP-B may also preferably be determined. Amino acid sequences comprising amino acids 1 to 381 for proSP-B are disclosed in Johansson 1992, FEBS Lett. 301:165-167, Jacobs 1987, J Biol Chem 262(20): 9808-11 and Jacobs 1988, J Biol Chem 263(2): 1093, or Pilot-Matias 1989, DNA 8:75-86 and are deposited in UniProtKB/Swiss-Prot data base under accession numbers P07988; Q96R04 or Genbank accession number P07988.3. The term also encompasses variants of the aforementioned specific SP-B peptides. Such variants have at least the same essential biological and immunological properties as the specific SP-B peptides. In particular, they share the same essential biological and immunological properties if they are detectable by the same specific assays referred to in this specification, e.g., by ELISA assays using polyclonal or monoclonal antibodies specifically recognizing the said SP-B peptides. A preferred assay is described in the accompanying Examples. Moreover, it is to be understood that a variant as referred to in accordance with the present invention shall have an amino acid sequence which differs due to at least one amino acid substitution, deletion and/or addition wherein the amino acid sequence of the variant is still, preferably, at least 50%, 60%, 70%, 80%, 85%, 90%, 92%, 95%, 97%, 98%, or 99% identical with the amino sequence of the specific SP-B peptides. The degree of identity between two amino acid sequences can be determined by algorithms well known in the art. Preferably, the degree of identity is to be determined by comparing two optimally aligned sequences over a comparison window, where the fragment of amino acid sequence in the comparison window may comprise additions or deletions (e.g., gaps or overhangs) as compared to the reference sequence (which does not comprise additions or deletions) for optimal alignment. The comparison window, preferably, is the entire length of the query sequence or at least 50% of its length. The percentage is calculated by determining the number of positions at which the identical amino acid residue occurs in both sequences to yield the number of matched positions, dividing the number of matched positions by the total number of positions in the window of comparison and multiplying the result by 100 to yield the percentage of sequence identity. Optimal alignment of sequences for comparison may be conducted by the local homology algorithm of Smith 1981, Add. APL. Math. 2:482, by the homology alignment algorithm of Needleman 1970, J. Mol. Biol. 48:443, by the search for similarity method of Pearson 1988, Proc. Natl. Acad Sci. (USA) 85: 2444 (1988), by computerized implementations of these algorithms (GAP, BESTFIT, BLAST, PASTA, and TFASTA in the Wisconsin Genetics Software Package, Genetics Computer Group (GCG), 575 Science Dr., Madison, WI), or by visual inspection. Given that two sequences have been identified for comparison, GAP and BESTFIT are preferably employed to determine their optimal alignment and, thus, the degree of identity. Preferably, the default values of 5.00 for gap weight and 0.30 for gap weight length are used. Variants referred to above may be allelic variants or any other species specific homologs, paralogs, or orthologs. Moreover, the variants referred to herein include fragments of the specific SP-B peptides or the aforementioned types of variants as long as these fragments have the essential immunological and biological properties as referred to above. Such fragments may be, e.g., degradation products of the SP-B peptides. Further included are variants which differ due to posttranslational modifications such as phosphorylation or myristylation. Moreover, the aforementioned SP-B peptides may be present as a monomer and/or in dimerized form.

Determining the amount of a SP-B peptide or any other peptide or polypeptide referred to in this specification relates to measuring the amount or concentration, preferably semi-quantitatively or quantitatively. Measuring can be done directly or indirectly. Direct measuring relates to measuring the amount or concentration of the peptide or polypeptide based on a signal which is obtained from the peptide or polypeptide itself and the intensity of which directly correlates with the number of molecules of the peptide present in the sample. Such a signal - sometimes referred to herein as intensity signal -may be obtained, e.g., by measuring an intensity value of a specific physical or chemical property of the peptide or polypeptide. Indirect measuring includes measuring of a signal obtained from a secondary component (i.e. a component not being the peptide or polypeptide itself) or a biological read out system, e.g., measurable cellular responses, ligands, labels, or enzymatic reaction products.

In accordance with the present invention, determining the amount of a peptide or polypeptide can be achieved by all known means for determining the amount of a peptide in a sample. Said means comprise immunoassay devices and methods which may utilize labelled molecules in various sandwich, competition, or other assay formats. Said assays will develop a signal which is indicative for the presence or absence of the peptide or polypeptide. Moreover, the signal strength can, preferably, be correlated directly or indirectly (e.g. reverse-proportional) to the amount of polypeptide present in a sample. Further suitable methods comprise measuring a physical or chemical property specific for the peptide or polypeptide such as its precise molecular mass or NMR spectrum. Said methods comprise, preferably, biosensors, optical devices coupled to immunoassays, biochips, analytical devices such as mass- spectrometers, NMR- analyzers, or chromatography devices. Further, methods include micro-plate ELISA-based methods, fully-automated or robotic immunoassays (available for example on Elecsys™ analyzers), CBA (an enzymatic Cobalt Binding Assay, available for example on Roche-Hitachi™ analyzers), and latex agglutination assays (available for example on Roche-Hitachi™ analyzers).

Preferably, determining the amount of a peptide or polypeptide comprises the steps of (a) contacting a cell capable of eliciting a cellular response the intensity of which is indicative of the amount of the peptide or polypeptide with the said peptide or polypeptide for an adequate period of time, (b) measuring the cellular response. For measuring cellular responses, the sample or processed sample is, preferably, added to a cell culture and an internal or external cellular response is measured. The cellular response may include the measurable expression of a reporter gene or the secretion of a substance, e.g. a peptide, polypeptide, or a small molecule. The expression or substance shall generate an intensity signal which correlates to the amount of the peptide or polypeptide.

Also preferably, determining the amount of a peptide or polypeptide comprises the step of measuring a specific intensity signal obtainable from the peptide or polypeptide in the sample. As described above, such a signal may be the signal intensity observed at an m/z variable specific for the peptide or polypeptide observed in mass spectra or a NMR spectrum specific for the peptide or polypeptide.

Determining the amount of a peptide or polypeptide may, preferably, comprises the steps of (a) contacting the peptide with a specific ligand, (b) preferably, removing non-bound ligand and other components which may be present in the sample, (c) measuring the amount of bound ligand, i.e. the complex of the peptide and the ligand formed in step (a). The bound ligand, i.e. the ligand or the ligand/peptide complex, will generate an intensity signal which reflects the amount of peptide or polypeptide originally present in the sample. Binding according to the present invention includes both covalent and non-covalent binding. A ligand according to the present invention can be any compound, e.g., a peptide, polypeptide, nucleic acid, or small molecule, binding to the peptide or polypeptide described herein. Preferred ligands include antibodies, nucleic acids, peptides or polypeptides such as receptors or binding partners for the peptide or polypeptide and fragments thereof comprising the binding domains for the peptides, and aptamers, e.g. nucleic acid or peptide aptamers. Methods to prepare such ligands are well-known in the art. For example, identification and production of suitable antibodies or aptamers is also offered by commercial suppliers. The person skilled in the art is familiar with methods to develop derivatives of such ligands with higher affinity or specificity. For example, random mutations can be introduced into the nucleic acids, peptides or polypeptides. These derivatives can then be tested for binding according to screening procedures known in the art, e.g. phage display. Antibodies as referred to herein include both polyclonal and monoclonal antibodies, as well as fragments thereof, such as Fv, Fab and F(ab)₂ fragments that are capable of binding antigen or hapten. The present invention also includes single chain antibodies and humanized hybrid antibodies wherein amino acid sequences of a non-human donor antibody exhibiting a desired antigen-specificity are combined with sequences of a human acceptor antibody. The donor sequences will usually include at least the antigen-binding amino acid residues of the donor but may comprise other structurally and/or functionally relevant amino acid residues of the donor antibody as well. Such hybrids can be prepared by several methods well known in the art. Preferably, the ligand or agent binds specifically to the peptide or polypeptide. Specific binding according to the present invention means that the ligand or agent should not bind substantially to, i.e. cross-react with, another peptide, polypeptide or substance present in the sample to be analysed. Preferably, the specifically bound peptide or polypeptide should be bound with at least 3 times higher, more preferably at least 10 times higher and even more preferably at least 50 times higher affinity than any other relevant peptide or polypeptide. Non-specific binding may be tolerable, if it can still be distinguished and measured unequivocally, e.g. according to its size on a Western Blot, or by its relatively higher abundance in the sample. Binding of the ligand can be measured by any method known in the art. Preferably, said method is semiquantitative or quantitative.

Binding of a ligand may be measured directly, e.g. by NMR or surface plasmon resonance. Second, if the ligand also serves as a substrate of an enzymatic activity of the peptide or polypeptide of interest, an enzymatic reaction product may be measured (e.g. the amount of a protease can be measured by measuring the amount of cleaved substrate, e.g. on a Western Blot). Alternatively, the ligand may exhibit enzymatic properties itself and the "ligand/peptide or polypeptide" complex or the ligand which was bound by the peptide or polypeptide, respectively, may be contacted with a suitable substrate allowing detection by the generation of an intensity signal. For measurement of enzymatic reaction products, preferably the amount of substrate is saturating. The substrate may also be labelled with a detectable label prior to the reaction. Preferably, the sample is contacted with the substrate for an adequate period of time. An adequate period of time refers to the time necessary for an detectable, preferably measurable, amount of product to be produced. Instead of measuring the amount of product, the time necessary for appearance of a given (e.g. detectable) amount of product can be measured. Third, the ligand may be coupled covalently or non-covalently to a label allowing detection and measurement of the ligand. Labelling may be done by direct or indirect methods. Direct labelling involves coupling of the label directly (covalently or non-covalently) to the ligand. Indirect labelling involves binding (covalently or non-covalently) of a secondary ligand to the first ligand. The secondary ligand should specifically bind to the first ligand. Said secondary ligand may be coupled with a suitable label and/or be the target (receptor) of tertiary ligand binding to the secondary ligand. The use of secondary, tertiary or even higher order ligands is often used to increase the signal. Suitable secondary and higher order ligands may include antibodies, secondary antibodies, and the well-known streptavidinbiotin system (Vector Laboratories, Inc.). The ligand or substrate may also be "tagged" with one or more tags as known in the art. Such tags may then be targets for higher order ligands. Suitable tags include biotin, digoxygenin, His-Tag, Glutathion-S-Transferase, FLAG, GFP, myc-tag, influenza A virus haemagglutinin (HA), maltose binding protein, and the like. In the case of a peptide or polypeptide, the tag is preferably at the N-terminus and/or C-terminus. Suitable labels are any labels detectable by an appropriate detection method. Typical labels include gold particles, latex beads, acridan ester, luminol, ruthenium, enzymatically active labels, radioactive labels, magnetic labels ("e.g. magnetic beads", including paramagnetic and superparamagnetic labels), and fluorescent labels. Enzymatically active labels include e.g. horseradish peroxidase, alkaline phosphatase, beta-Galactosidase, Luciferase, and derivatives thereof. Suitable substrates for detection include di-amino-benzidine (DAB), 3,3'-5,5'-tetramethylbenzidine, NBT-BCIP (4-nitro blue tetrazolium chloride and 5-bromo-4-chloro-3-indolyl-phosphate, available as ready-made stock solution from Roche Diagnostics), CDP-Star™ (Amersham Biosciences), ECF™ (Amersham Biosciences). A suitable enzyme-substrate combination may result in a coloured reaction product, fluorescence or chemiluminescence, which can be measured according to methods known in the art (e.g. using a light-sensitive film or a suitable camera system). As for measuring the enyzmatic reaction, the criteria given above apply analogously. Typical fluorescent labels include fluorescent proteins (such as GFP and its derivatives), Cy3, Cy5, Texas Red, Fluorescein, and the Alexa dyes (e.g. Alexa 568). Further fluorescent labels are available e.g. from Molecular Probes (Oregon). Also the use of quantum dots as fluorescent labels is contemplated. Typical radioactive labels include ³⁵S, ¹²⁵I, ³²P, ³³P and the like. A radioactive label can be detected by any method known and appropriate, e.g. a light-sensitive film or a phosphor imager. Suitable measurement methods according the present invention also include precipitation (particularly immunoprecipitation), electrochemiluminescence (electro-generated chemiluminescence), RIA (radioimmunoassay), ELISA (enzyme-linked immunosorbent assay), sandwich enzyme immune tests, electrochemiluminescence sandwich immunoassays (ECLIA), dissociation-enhanced lanthanide fluoro immuno assay (DELFIA), scintillation proximity assay (SPA), turbidimetry, nephelometry, latex-enhanced turbidimetry or nephelometry, or solid phase immune tests. Further methods known in the art (such as gel electrophoresis, 2D gel electrophoresis, SDS polyacrylamid gel electrophoresis (SDS-PAGE), Western Blotting, and mass spectrometry), can be used alone or in combination with labelling or other detection methods as described above.

Further suitable techniques for the determination of a polypeptide or peptide are described in the following.

The amount of a peptide or polypeptide may be, also preferably, determined as follows: (a) contacting a solid support comprising a ligand for the peptide or polypeptide as specified above with a sample comprising the peptide or polypeptide, (b) preferably, removing unbound peptide or polypeptide as well as remaining sample material and (c) measuring the amount peptide or polypeptide which is bound to the support. Preferably, the amount of the complex of the ligand and the peptide or polypeptide formed on the solid support is measured. It will be understood that the amount of the complex formed during the determination shall represent the amount of the peptide or polypeptide originally present in the sample. The ligand is, preferably chosen from the group consisting of nucleic acids, peptides, polypeptides, antibodies and aptamers and is, preferably, present on a solid support in immobilized form. Materials for manufacturing solid supports are well known in the art and include, inter alia, commercially available column materials, polystyrene beads, latex beads, magnetic beads, colloid metal particles, glass and/or silicon chips and surfaces, nitrocellulose strips, membranes, sheets, duracytes, wells and walls of reaction trays, plastic tubes etc. The ligand or agent may be bound to many different carriers. Examples of well-known carriers include glass, polystyrene, polyvinyl chloride, polypropylene, polyethylene, polycarbonate, dextran, nylon, amyloses, natural and modified celluloses, polyacrylamides, agaroses, and magnetite. The nature of the carrier can be either soluble or insoluble for the purposes of the invention. Suitable methods for fixing/immobilizing said ligand are well known and include, but are not limited to ionic, hydrophobic, covalent interactions and the like. It is also contemplated to use "suspension arrays" as arrays according to the present invention (Nolan 2002, Trends Biotechnol. 20(1):9-12). In such suspension arrays, the carrier, e.g. a microbead or microsphere, is present in suspension. The array consists of different microbeads or microspheres, possibly labelled, carrying different ligands. Methods of producing such arrays, for example based on solid-phase chemistry and photo-labile protective groups, are generally known (US 5,744,305).

The term "amount" as used herein encompasses the absolute amount of a polypeptide or peptide, the relative amount or concentration of the said polypeptide or peptide as well as any value or parameter which correlates thereto or can be derived therefrom. Such values or parameters comprise intensity signal values from all specific physical or chemical properties obtained from the said peptides by direct measurements, e.g., intensity values in mass spectra or NMR spectra. Moreover, encompassed are all values or parameters which are obtained by indirect measurements specified elsewhere in this description, e.g., response levels determined from biological read out systems in response to the peptides or intensity signals obtained from specifically bound ligands. It is to be understood that values correlating to the aforementioned amounts or parameters can also be obtained by all standard mathematical operations.

The term "comparing" as used herein encompasses comparing the amount of the peptide or polypeptide comprised by the sample to be analysed with an amount of a suitable reference source specified elsewhere in this description. It is to be understood that comparing as used herein refers to a comparison of corresponding parameters or values, e.g., an absolute amount is compared to an absolute reference amount while a concentration is compared to a reference concentration or an intensity signal obtained from a test sample is compared to the same type of intensity signal of a reference sample. The comparison referred to in step (b) of the method of the present invention may be carried out manually or computer assisted. For a computer assisted comparison, the value of the determined amount may be compared to values corresponding to suitable references which are stored in a database by a computer program. The computer program may further evaluate the result of the comparison, i.e. automatically provide the desired assessment in a suitable output format. Based on the comparison of the amount determined in step a) and the reference amount, it is possible to assess whether a subject exhibiting a symptom of an acute cardiovascular event suffers from pulmonary complication, or not. Therefore, the reference amount is to be chosen so that either a difference or a similarity in the compared amounts allows identifying those test subjects which belong into the group of subjects exhibiting a symptom of an acute cardiovascular event and suffering from pulmonary complication.

Accordingly, the term "reference amount" as used herein refers to an amount which allows assessing whether a subject suffering from pulmonary hypertension has alveolar damage, or not. Accordingly, the reference may, e.g., be derived from (i) a subject or group of subjects suffering from pulmonary hypertension and having alveolar damage, (ii) a subject or group of subjects suffering from pulmonary hypertension and not having alveolar damage or (iii) a clinically apparently healthy subject or a group of such subjects. The reference amount may be used to define and establish a threshold amount. The threshold amount, preferably, allows for a rule-in and/or a rule-out diagnosis. The reference amount applicable for an individual subject may vary depending on various physiological parameters such as age, gender, or subpopulation, as well as on the means used for the determination of the polypeptide or peptide referred to herein. A suitable reference amount may be determined from a reference sample to be analysed together, i.e. simultaneously or subsequently, with the test sample.
In one embodiment of the method of the present invention, said reference amount is derived from a subject or a group of subjects suffering from pulmonary hypertension known to have alveolar damage and wherein an essentially identical or increased amount of the SP-B peptide is indicative for a subject having alveolar damage, whereas a decreased amount of the SP-B peptide is indicative for a subject not having alveolar damage.

In yet another embodiment of the method of the present invention, said reference amount is derived from a subject or a group of subjects suffering from pulmonary hypertension known not to have alveolar damage and wherein an essentially identical or decreased amount of the SP-B peptide is indicative for a subject not having alveolar damage, whereas a increased amount of the SP-B peptide is indicative for a subject having alveolar damage.

Reference amounts can be calculated for a cohort of subjects (i.e. (i) a subject or group of subjects suffering from pulmonary hypertension and having alveolar damage, (ii) a subject or group of subjects suffering from pulmonary hypertension and not having alveolar damage or (iii) a clinically apparently healthy subject or a group of such subjects) based on the average or mean values for a given biomarker by applying standard statistically methods. In particular, accuracy of a test such as a method aiming to diagnose an event, or not, is best described by its receiver-operating characteristics (ROC) (see, e.g., Zweig 1993, Clin. Chem. 39:561-577). The ROC graph is a plot of all of the sensitivity/specificity pairs resulting from continuously varying the decision threshold over the entire range of data observed. The clinical performance of a diagnostic method depends on its accuracy, i.e. its ability to correctly allocate subjects to a certain prognosis or diagnosis. The ROC plot indicates the overlap between the two distributions by plotting the sensitivity versus 1-specificity for the complete range of thresholds suitable for making a distinction. On the y-axis is sensitivity, or the true-positive fraction which is defined as the ratio of number of true-positive test results to the product of number of true-positive and number of false-negative test results. This has also been referred to as positivity in the presence of a disease or condition. It is calculated solely from the affected subgroup. On the x-axis is the false-positive fraction, or 1-specificity which is defined as the ratio of number of false-positive results to the product of number of true-negative and number of false-positive results. It is an index of specificity and is calculated entirely from the unaffected subgroup. Because the true- and false-positive fractions are calculated entirely separately, by using the test results from two different subgroups, the ROC plot is independent of the prevalence of the event in the cohort. Each point on the ROC plot represents a sensitivity/-specificity pair corresponding to a particular decision threshold. A test with perfect discrimination (no overlap in the two distributions of results) has an ROC plot that passes through the upper left corner, where the true-positive fraction is 1.0, or 100% (perfect sensitivity), and the false-positive fraction is 0 (perfect specificity). The theoretical plot for a test with no discrimination (identical distributions of results for the two groups) is a 45° diagonal line from the lower left corner to the upper right corner. Most plots fall in between these two extremes. (If the ROC plot falls completely below the 45° diagonal, this is easily remedied by reversing the criterion for "positivity" from "greater than" to "less than" or vice versa.) Qualitatively, the closer the plot is to the upper left corner, the higher the overall accuracy of the test. Dependent on a desired confidence interval, a threshold can be derived from the ROC curve allowing for the diagnosis or prediction for a given event with a proper balance of sensitivity and specificity, respectively. Accordingly, the reference to be used for the aforementioned method of the present invention, i.e. a threshold which allows to discriminate between subjects suffering from a pulmonary hypertension having, or not, alveolar damage can be generated, preferably, by establishing a ROC for said cohort as described above and deriving a threshold amount therefrom.

Preferably, the median values or percentiles for a biomarker determined in a reference population as specified above may be also used as a basis for establishing thresholds. Suitable median values or values for the 25^{th} and 75^{th} percentiles are disclosed in the accompanying Examples, below. More preferably, the 75^{th} percentile values for proSP-B of about 40 to 55 ng/ml found in coronary artery disease subjects having essentially normal cardiac function, i.e. normal or nearly normal cardiac function, (about 40 ng/ml) or coronary artery disease subjects having abnormal cardiac function without clinically apparent reduced left ventricular ejection fraction (about 55 ng/ml) was found to be a threshold for differentiating between subjects suffering from pulmonary hypertension and having alveolar damage, or not. More preferably, the 75^{th} percentile values for C-terminal fragment of proSP-B of about 79 to 105 ng/ml found in coronary artery disease subjects having normal cardiac function (about 79 ng/ml) or coronary artery disease subjects having abnormal cardiac function without clinically apparent reduced left ventricular ejection fraction (about 105 ng/ml) could be used be used for establishing a preferred threshold.

In principle, it has been found in accordance with the present invention that the extent oft he alveolar damage correlates with the amount oft he SP-B peptide which is determined. Accordingly, an amount of SP-B which is between about 1 times and about 2 times the threshold amount shall be indicative for a less extensive alveolar damage, whereas an amount of the SP-B peptide which is above about 2 times the threshold shall be indicative for a more extensive alveolar damage.

"About" as used in accordance with the present invention means +/- 20%, +/- 10%, +/- 5%, +-2 % or +-/ 1% from the said value.

Preferably, the cause of alveolar damage can also be diagnosed by the method of the present invention as well. The alveolar damage may, in principle, be caused by alveolar hypoxia or left ventricular dysfunction. In the latter case, left heart failure could be the cause of the alveolar damage, i.e. the alveolar damage results via, e.g., pulmonary artery hypertension, from heart failure, whereas in the other case, the alveolar damage directly accompanies alveolar hypoxia. It will be understood by those skilled in the art that the cause of the alveolar damage has therapeutic implications. If alveolar hypoxia is diagnosed as the cause of the alveolar damage, monitoring and therapy shall concentrate on the said alveolar hypoxia. If left heart failure is diagnosed as the cause of the alveolar damage, left heart failure therapy and monitoring shall be applied.

Thus, in a preferred embodiment of the method of the present invention, said method further comprises determining the amount of a natriuretic peptide and/or a cardiac troponin in said sample and comparing said amount(s) to a reference amount whereby it is diagnosed whether the said subject also exhibits left ventricular dysfunction, or not. Preferably, the diagnosis of alveolar damage and a left ventricular dysfunction is indicative for left heart failure.

The reference amounts for the natriuretic peptide and/or cardiac troponin in this case are, preferably, derived from a subject or group of subjects suffering from heart failure and a left ventricular dysfunction which is clinically apparent by a statistically significant reduced left ventricular ejection fraction. A determined amount for the natriuretic peptide and/or the cardiac troponin is identical or larger than the reference amount is, preferably, indicative for left ventricular dysfunction in this case while an amount which is decreased compared to the reference amount is indicative for the absence of a left ventricular dysfunction. Preferably, the median values or percentiles for said biomarkers determined in a reference population as specified above may also be also used as a basis for establishing thresholds. Suitable median values or values for the 25^{th} and 75^{th} percentiles are disclosed in the accompanying Examples, below. More preferably, the 75^{th} percentile values for a natriuretic peptide, i.e. NT-proBNP of about 984 pg/ml found in subjects not suffering from a left ventricular systolic dysfunction can be applied as thresholds. More preferably, the 75^{th} percentile values for a cardiac troponin, i.e. troponin T of about 17 pg/ml found in subjects not suffering from a left ventricular systolic dysfunction can be applied as thresholds.

The term "natriuretic peptide" comprises Atrial Natriuretic Peptide (ANP)-type and Brain Natriuretic Peptide (BNP)-type peptides and variants thereof having the same diagnostic potential (see e.g. Bonow, 1996, Circulation 93: 1946-1950). ANP-type peptides comprise pre-proANP, proANP, NT-proANP, and ANP. BNP-type peptides comprise pre-proBNP, proBNP, NT-proBNP, and BNP. The pre-pro peptide (134 amino acids in the case of pre-proBNP) comprises a short signal peptide, which is enzymatically cleaved off to release the pro peptide (108 amino acids in the case of proBNP). The pro peptide is further cleaved into an N-terminal pro peptide (NT-pro peptide, 76 amino acids in case of NT-proBNP) and the active hormone (32 amino acids in the case of BNP, 28 amino acids in the case of ANP). Preferred natriuretic peptides according to the present invention are NT-proANP, ANP, NT-proBNP, BNP, and variants thereof. ANP and BNP are the active hormones and have a shorter half-life than their respective inactive counterparts, NT-proANP and NT-proBNP. BNP is metabolised in the blood, whereas NT-proBNP circulates in the blood as an intact molecule and as such is eliminated renally. The in-vivo half-life of NTproBNP is 120 min longer than that of BNP, which is 20 min (Smith 2000, J Endocrinol. 167: 239-46.). Preanalytics are more robust with NT-proBNP allowing easy transportation of the sample to a central laboratory (Mueller 2004, Clin Chem Lab Med 42: 942-4.). Blood samples can be stored at room temperature for several days or may be mailed or shipped without recovery loss. In contrast, storage of BNP for 48 hours at room temperature or at 4° Celsius leads to a concentration loss of at least 20 % (Mueller loc.cit.; Wu 2004, Clin Chem 50: 867-73.). Therefore, depending on the time-course or properties of interest, either measurement of the active or the inactive forms of the natriuretic peptide can be advantageous. The most preferred natriuretic peptides according to the present invention are NT-proBNP and variants thereof. As briefly discussed above, the human NT-proBNP, as referred to in accordance with the present invention, is a polypeptide comprising, preferably, 76 amino acids in length corresponding to the N-terminal portion of the human NT-proBNP molecule. The structure of the human BNP and NT-proBNP has been described already in detail in the prior art, e.g., WO 02/089657, WO 02/083913 or Bonow loc. cit. Preferably, human NT-proBNP as used herein is human NT-proBNP as disclosed in EP 0 648 228 B1. These prior art documents are herewith incorporated by reference with respect to the specific sequences of NT-proBNP and variants thereof disclosed therein. The NT-proBNP referred to in accordance with the present invention further encompasses allelic and other variants of said specific sequence for human NT-proBNP discussed above. Specifically, envisaged are variant polypeptides which are on the amino acid level at least 60 % identical, more preferably at least 70 %, at least 80 %, at least 90 %, at least 95 %, at least 98% or at least 99 % identical, to human NT-proBNP. The degree of identity between two amino acid sequences, in principle, can be determined by algorithms described elsewhere herein. Also encompassed are variant polypeptides having amino acid deletions, substitutions, and/or additions compared to the amino acid sequence of human NT-proBNP as long as the said polypeptides have NT-proBNP properties. NT-proBNP properties as referred to herein are immunological and/or biological properties. Preferably, the NT-proBNP variants have immunological properties (i.e. epitope composition) comparable to those of NT-proBNP. Thus, the variants shall be recognizable by the aforementioned means or ligands used for determination of the amount of the natriuretic peptides. Biological and/or immunological NT-proBNP properties can be detected by the assay described in Karl et al. (Karl 1999, Scand J Clin Invest 59:177-181), Yeo et al. (Yeo 2003, Clinica Chimica Acta 338:107-115). Variants also include posttranslationally modified peptides such as glycosylated or myristylated peptides. Further, a variant in accordance with the present invention is also a peptide or polypeptide which has been modified after collection of the sample, for example by covalent or non-covalent attachment of a label, particularly a radioactive or fluorescent label, to the peptide.

The term "cardiac troponin" refers to all troponin isoforms expressed in cells of the heart and, preferably, the subendocardial cells. These isoforms are well characterized in the art as described, e.g., in Anderson 1995, Circulation Research, vol. 76, no. 4: 681-686 and Ferrieres 1998, Clinical Chemistry, 44: 487-493. Preferably, cardiac troponin refers to troponin T and/or troponin I, and, most preferably, to troponin T. It is to be understood that isoforms of troponins may be determined in the method of the present invention together, i.e. simultaneously or sequentially, or individually, i.e. without determining the other isoform at all. Amino acid sequences for human troponin T and human troponin I are disclosed in Anderson, loc cit and Ferrieres 1998, Clinical Chemistry, 44: 487-493. The term "cardiac troponin" encompasses also variants of the aforementioned specific troponins, i.e., preferably, of tropoinin T or troponin I. Such variants have at least the same essential biological and immunological properties as the specific cardiac troponins. In particular, they share the same essential biological and immunological properties if they are detectable by the same specific assays referred to in this specification, e.g., by ELISA Assays using polyclonal or monoclonal antibodies specifically recognizing the said cardiac troponins. Moreover, it is to be understood that a variant as referred to in accordance with the present invention shall have an amino acid sequence which differs due to at least one amino acid substitution, deletion and/or addition wherein the amino acid sequence of the variant is still, preferably, at least 50%, 60%, 70%, 80%, 85%, 90%, 92%, 95%, 97%, 98%, or 99% identical with the amino sequence of the specific troponin. The degree of identity between two amino acid sequences, in principle, can be determined by algorithms described elsewhere herein. Variants may be allelic variants or any other species specific homologs, paralogs, or orthologs. Moreover, the variants referred to herein include fragments of the specific cardiac troponins or the aforementioned types of variants as long as these fragments have the essential immunological and biological properties as referred to above. Such fragments may be, e.g., degradation products of the troponins. Further included are variants which differ due to posttranslational modifications such as phosphorylation or myristylation.

Moreover, in a preferred embodiment of the method of the present invention, said method further comprises assessing an echocardiogram of said patient for left ventricular dysfunction. Preferably, the diagnosis of alveolar damage and a left ventricular dysfunction is indicative for left heart failure. Moreover, in addition to or instead of the echocardiogram, CT, optical coherence tomography (OCT) or MRI images may be assessed in the method of the invention for left ventricular dysfunction. Further, the wedge pressure may be taken into account as well.

Advantageously, it has been found in the studies underlying the present invention that SP-B peptides, such as proSP-B or the C-terminal fragment of proSP-B, in a body fluid such as blood, plasma or serum can serve as a biomarker that allows for identifying an alveolar damage in pulmonary hypertension subjects. Thanks to the present invention, it is possible to identify those patients with alveolar damage such that they can be monitored subjected to an individual therapy according to their needs. Moreover, if combined with the assessment of left ventricular dysfunction, it can be discriminated between subjects having alveolar hypoxia as the cause of the alveolar damage or subjects suffering from heart failure, in particular left hart failure, as the cause of the alveolar damage. Thus, monitoring and therapy can be individually adapted to either hypoxia therapy and monitoring or heart failure monitoring an therapy.

In an aspect of the invention, a method for establishing an aid for diagnosing whether a subject suffering from pulmonary hypertension having alveolar damage, or not, is contemplated, said method comprising:
a) determining the amount of a SP-B peptide in a sample of said subject, said determining comprises (i) bringing the sample into contact with a detection agent that specifically binds to the SP-B peptide for a time sufficient to allow for the formation of a complex of the said detection agent and the SP-B peptide from the sample, (ii) measuring the amount of the formed complex, wherein the said amount of the formed complex is proportional to the amount of the SP-B peptide present in the sample, and (iii) transforming the amount of the formed complex into an amount of the SP-B peptide reflecting the amount of the SP-B peptide present in the sample;
b) comparing said amount to a reference; and
c) establishing an aid for diagnosing alveolar damage based on the result of the comparison made in step b).

A suitable detection agent may be, in an aspect, an antibody which is specifically binds to a SP-B peptide, i.e. mature SP-B, proSP-B or the C-terminal fragment of SP-B, in a sample of a subject to be investigated by the method of the invention. Another detection agent that can be applied, in an aspect, may be an aptamere which specifically binds to the SP-B peptide in the sample. In yet an aspect the, sample is removed from the complex formed between the detection agent and the SP-B peptide prior to the measurement of the amount of formed complex. Accordingly, in an aspect, the detection agent may be immobilized on a solid support. In yet an aspect, the sample can be removed from the formed complex on the solid support by applying a washing solution. The formed complex shall be proportional to the amount of the SP-B peptide present in the sample. It will be understood that the specificity and/or sensitivity of the detection agent to be applied defines the degree of proportion of SP-B peptide comprised in the sample which is capable of being specifically bound. Further details on how the determination can be carried out are also found elsewhere herein. The amount of formed complex shall be transformed into an amount of SP-B peptide reflecting the amount indeed present in the sample. Such an amount, in an aspect, may be essentially the amount present in the sample or may be, in another aspect, an amount which is a certain proportion thereof due to the relationship between the formed complex and the amount present in the original sample.

In yet an aspect of the aforementioned method, step a) may be carried out by an analyzing unit, in an aspect, an analyzing unit as defined elsewhere herein.

In an aspect of the method of the invention, the amount determined in step a) is compared to a reference. In an aspect, the reference is a reference as defined elsewhere herein. In yet another aspect, the reference takes into account the proportional relationship between the measured amount of complex and the amount present in the original sample. Thus, the references applied in an aspect of the method of the invention are artificial references which are adopted to reflect the limitations of the detection agent that has been used. In another aspect, said relationship can be also taken into account when carrying out the comparison, e.g., by including a normalization and/or correction calculation step for the determined amount prior to actually comparing the value of the determined amount and the reference. Again, the normalization and/or correction calculation step for the determined amount adopts the comparison step such that the limitations of the detection agent that has been used are reflected properly. In an aspect, the comparison is carried out automatically, e.g., assisted by a computer system or the like.

The aid for diagnosing is established based on the comparison carried out in step b) by allocating the subject either into a group of subjects suffering from a pulmonary hypertension having alveolar damage or not having alveolar damage As discussed elsewhere herein already, the allocation of the investigated subject must not be correct in 100% of the investigated cases. Moreover, the groups of subjects into which the investigated subject is allocated are artificial groups in that they are established based on statistical considerations, i.e. a certain preselected degree of likelihood based on which the method of the invention shall operate. Thus, the method may establish an aid of diagnosis which may, in an aspect, require further strengthening of the diagnosis by other techniques. In an aspect of the invention, the aid for diagnosing is established automatically, e.g., assisted by a computer system or the like.

In an aspect of the method of the invention, said method further comprises a step of recommending and/or managing the subject according to the result of the aid of diagnosis established in step c). Such a recommendation may, in an aspect, be an adaptation of life style, nutrition and the like aiming to improve the life circumstances, the application of therapeutic measures, e.g., hypoxia or heart failure therapy, as set forth elsewhere herein in detail, and/or adapting intensiveness of disease monitoring.

In an aspect of the aforementioned method, steps b) and/or c) are carried out by an evaluation unit as set forth elsewhere herein.

It is to be understood that the definitions and explanations of the terms made above and below apply accordingly for all embodiments described in this specification and the accompanying claims.

In a preferred embodiment of the method of the present invention, said method further comprises recommending a therapy for treating pulmonary hypertension associated with the said alveolar damage.

The term "therapy for treating pulmonary hypertension associated with the said alveolar damage" refers to any therapy improving or curing alveolar damage or preventing worsening thereof. Dependent on the cause of the alveolar damage, said therapy may be a therapy of alveolar hypoxia and/or a therapy of heart failure. Preferably, the said therapy for treating alveolar damage encompasses the administration of a drug selected from the group consisting of: Prostaglandins, calcium channel blockers, prostacyclins, such as epoprostenol, treproatinil, iloprost, or beraprost, ET1 receptor blockers, such as bosentan or ambrisentan, immunosuppressive agents, such as cyclophosphamide, nitric oxide and soluble guanylate cyclase stimulators, such as riociguat, Rho kinase inhibitors, such as fasudil, PDGF receptor inhibitors, such as imatinib, beta blockers, angiotensin receptor blockers, ACE inhibitors, phosphodiesterase inhibitors, preferably, sildenafil, nitrates, and diuretics, preferably, short acting loop diuretics and long acting thiazides, aldosterone antagonists (for details, see also Fukumoto loc cit., Mathew loc. Cit.).

In particular, if left heart failure is diagnosed as the cause of alveolar damage, the therapy shall be selected from calcium channel blockers, prostacyclins, such as epoprostenol, treproatinil, iloprost, or beraprost, nitric oxide and soluble guanylate cyclase stimulators, such as riociguat, beta blockers, angiotensin receptor blockers, ACE inhibitors, phosphodiesterase inhibitors, preferably, sildenafil, and diuretics, preferably, short acting loop diuretics and long acting thiazides, aldosterone antagonists.

In particular, if alveolar hypoxia is diagnosed as the cause of alveolar damage, the therapy shall be selected from Prostaglandins, ET1 receptor blockers, such as bosentan or ambrisentan, immunosuppressive agents, such as cyclophosphamide, Rho kinase inhibitors, such as fasudil, and PDGF receptor inhibitors, such as imatinib.

In another preferred embodiment of the method of the present invention, said method further comprises recommending a monitoring intensiveness.

The term "monitoring intensiveness" as used herein refers to the frequency of monitoring (e.g., weak, regular or close monitoring) and to the extent of investigation carried out per monitoring (e.g. additional application of echocardiography and/or other imaging techniques referred to elsewhere herein, or not, or additional measurement peripheral hypoxia markers (pO₂ or pCO₂), or not).

Moreover, the monitoring may include monitoring of whether a therapy is successful. In particular, the monitoring may encompass monitoring whether left heart failure therapy, e.g., as set forth above, improves (i.e. cures or ameliorates) the alveolar damage. Moreover, the monitoring may encompass monitoring whether alveolar hypoxia therapy, e.g., as set forth above, improves (i.e. cures or ameliorates) the alveolar damage. If no improvement is determined, the therapy may be adapted or replaced. Thus, the method could aid in treatment selection.

The present invention also relate to the use of a SP-B peptide or detection agents which specifically bind thereto in a sample of a subject exhibiting pulmonary hypertension for diagnosing alveolar damage. Moreover, also encompassed is the use of a SP-B peptide or detection agents which specifically bind thereto in a sample of a subject exhibiting pulmonary hypertension for the manufacture of a diagnostic or pharmaceutical composition for diagnosing alveolar damage.

The term "detection agent" as used herein refers to an agent that is capable of specifically recognizing and binding to the biomarker polypeptide(s) present in a sample. Moreover, the said agent shall allow for direct or indirect detection of the complex formed by the said agent and the biomarker. Direct detection can be achieved by including into the agent a detectable label. Indirect labelling may be achieved by a further agent that specifically binds to the complex comprising the biomarker and the detection agent wherein the said further agent is than capable of generating a detectable signal. Suitable compounds which can be used as detection agents are well known in the art. Preferably, the detection agent is an antibody or aptamere which specifically binds to the biomarker.
The present invention relates to a device adapted for carrying out the method of the invention comprising
a) an analyzing unit comprising a detection agent which specifically binds to a SP-B peptide, said unit being adapted for determining the amount of the SP-B peptide in a sample of a subject exhibiting pulmonary hypertension; and
b) an evaluation unit for comparing the determined amount with a reference amount whereby it can be diagnosed whether the said subject has alveolar damage, said unit comprising a database with reference amount values and a computer-implemented algorithm carrying out the comparison.

The term "device" as used herein relates to a system comprising the aforementioned units operatively linked to each other as to allow the diagnosis or monitoring according to the methods of the invention. Preferred detection agents which can be used for the analysing unit are disclosed elsewhere herein. The analysing unit, preferably, comprises said detection agents in immobilized form on a solid support which is to be contacted to the sample comprising the biomarkers the amount of which is to be determined. Moreover, the analysing unit can also comprise a detector which determines the amount of detection agent which is specifically bound to the biomarker(s). The determined amount can be transmitted to the evaluation unit. Said evaluation unit comprises a data processing element, such as a computer, with an implemented algorithm for carrying out a comparison between the determined amount and a suitable reference. Suitable references are either derived from a subject or group of subjects as defined above in context with the method of the present invention. The results may be given as output of parametric diagnostic raw data, preferably, as absolute or relative amounts. It is to be understood that these data will need interpretation by the clinician. However, also envisage are expert system devices wherein the output comprises processed diagnostic raw data the interpretation of which does not require a specialized clinician.

It follows from the above that according to some embodiments of the instant disclosure, portions of some steps of methods disclosed and described herein may be performed by a computing device. A computing device may be a general purpose computer or a portable computing device, for example. It should also be understood that multiple computing devices may be used together, such as over a network or other methods of transferring data, for performing one or more steps of the methods disclosed herein. Exemplary computing devices include desktop computers, laptop computers, personal data assistants ("PDA"), such as BLACKBERRY brand devices, cellular devices, tablet computers, servers, and the like. In general, a computing device comprises a processor capable of executing a plurality of instructions (such as a program of software).

A computing device has access to a memory. A memory is a computer readable medium and may comprise a single storage device or multiple storage devices, located either locally with the computing device or accessible to the computing device across a network, for example. Computer-readable media may be any available media that can be accessed by the computing device and includes both volatile and non-volatile media. Further, computer readable-media may be one or both of removable and non-removable media. By way of example, and not limitation, computer-readable media may comprise computer storage media. Exemplary computer storage media includes, but is not limited to, RAM, ROM, EEPROM, flash memory or any other memory technology, CD-ROM, Digital Versatile Disk (DVD) or other optical disk storage, magnetic cassettes, magnetic tape, magnetic disk storage or other magnetic storage devices, or any other medium which can be used for storing a plurality of instructions capable of being accessed by the computing device and executed by the processor of the computing device.

According to embodiments of the instant disclosure, software may include instructions which, when executed by a processor of the computing device, may perform one or more steps of the methods disclosed herein. Some of the instructions may be adapted to produce signals that control operation of other machines and thus may operate through those control signals to transform materials far removed from the computer itself. These descriptions and representations are the means used by those skilled in the art of data processing, for example, to most effectively convey the substance of their work to others skilled in the art.

The plurality of instructions may also comprise an algorithm which is generally conceived to be a self-consistent sequence of steps leading to a desired result. These steps are those requiring physical manipulations of physical quantities. Usually, though not necessarily, these quantities take the form of electrical or magnetic pulses or signals capable of being stored, transferred, transformed, combined, compared, and otherwise manipulated. It proves convenient at times, principally for reasons of common usage, to refer to these signals as values, characters, display data, numbers, or the like as a reference to the physical items or manifestations in which such signals are embodied or expressed. It should be borne in mind, however, that all of these and similar terms are to be associated with the appropriate physical quantities and are merely used here as convenient labels applied to these quantities.

The computing device may also have access to an output device. Exemplary output devices include fax machines, displays, printers, and files, for example. According to some embodiments of the present disclosure, a computing device may perform one or more steps of a method disclosed herein, and thereafter provide an output, via an output device, relating to a result, indication, ratio or other factor of the method.

The present invention also encompasses a kit adapted for carrying out the method of the invention comprising a detection agent for the SP-B peptide, reference standards as well as instructions for carrying out the said method.

The term "kit" as used herein refers to a collection of the aforementioned components, preferably, provided in separately or within a single container. The container also comprises instructions for carrying out the method of the present invention. These instructions may be in the form of a manual or may be provided by a computer program code which is capable of carrying out the comparisons referred to in the methods of the present invention and to establish a diagnosis accordingly when implemented on a computer or a data processing device. The computer program code may be provided on a data storage medium or device such as an optical storage medium (e.g., a Compact Disc) or directly on a computer or data processing device. Further, the kit shall comprise at least one standard for a reference as defined herein above, i.e. a solution with a pre-defined amount for the SP-B peptide polypeptide representing a reference amount. Such a standard may represent, e.g., the amount of SP-B peptide from a subject or group of subjects suffering from pulmonary hypertension and having alveolar damage or a subject or group of subjects suffering from pulmonary hypertension and not having alveolar damage or a clinically apparently healthy subject or group thereof.

The kit, preferably, also comprises detection agents for at least one further biomarker referred to herein and, more preferably, for a cardiac troponin and/or a natriuretic peptide, as well as reference standards for the at least one further biomarker.

In some embodiments, a kit disclosed herein includes at least one component or a packaged combination of components for practicing a disclosed method. By "packaged combination" it is meant that the kits provide a single package that contains a combination of one or more components, such as probes (for example, an antibody), controls, buffers, reagents (for example, conjugate and/or substrate) instructions, and the like, as disclosed herein. A kit containing a single container is also included within the definition of "packaged combination." In some embodiments, the kits include at least one probe, for example an antibody (having specific affinity for an epitope of a biomarker as disclosed herein. For example, the kits may include an antibody that is labelled with a fluorophore or an antibody that is a member of a fusion protein. In the kit, the probe may be immobilized, and may be immobilised in a specific conformation. For example, an immobilized probe may be provided in a kit to specifically bind target protein, to detect target protein in a sample, and/or to remove target protein from a sample.

According to some embodiments, kits include at least one probe, which may be immobilized, in at least one container. Kits may also include multiple probes, optionally immobilized, in one or more containers. For example, the multiple probes may be present in a single container or in separate containers, for example, wherein each container contains a single probe.

In some embodiments, a kit may include one or more non-immobilized probe and one or more solid support that does or does not include an immobilized probe. Some such embodiments may comprise some or all of the reagents and supplies needed for immobilizing one or more probes to the solid support, or some or all of the reagents and supplies needed for binding of immobilized probes to specific proteins within a sample.

In certain embodiments, a single probe (including multiple copies of the same probe) may be immobilized on a single solid support and provided in a single container. In other embodiments, two or more probes, each specific for a different target protein or a different form of a single target protein (such as a specific epitope), a provided in a single container. In some such embodiments, an immobilized probe may be provided in multiple different containers (e.g., in single-use form), or multiple immobilized probes may be provided in multiple different containers. In further embodiments, the probes may be immobilized on multiple different type of solid supports. Any combination of immobilized probe(s) and container(s) is contemplated for the kits disclosed herein, and any combination thereof may be selected to achieve a suitable kit for a desired use.

A container of the kits may be any container that is suitable for packaging and/or containing one or more components disclosed herein, including for example probes (for example, an antibody), controls, buffers, and reagents (for example, conjugate and/or substrate). Suitable materials include, but are not limited to, glass, plastic, cardboard or other paper product, wood, metal, and any alloy thereof. In some embodiments, the container may completely encase an immobilized probe(s) or may simply cover the probe to minimize contamination by dust, oils, etc., and expose to light. In some further embodiments, he kits may comprise a single container or multiple containers, and where multiple containers are present, each container may be the same as all other containers, different than others, or different than some but not all other containers.

All references cited in this specification are herewith incorporated by reference with respect to their entire disclosure content and the disclosure content specifically mentioned in this specification.

### EXAMPLES

The following Examples shall merely illustrate the invention. They shall, whatsoever, not be construed as limiting the scope.

### Example 1: Determination of biomarkers in serum samples

The proSP-B assay uses a mouse monoclonal anti-proSP-B (N-terminus) antibody as a capture and a mouse monoclonal anti-proSP-B (C-terminus) antibody as a detection reagent. The assay principle is a sandwich format. The antibody to the N-terminal pro-sequence binds to an epitope comprised in the peptide sequence ranging from amino acid 160 to 169 of proSP-B. The antibody to the C-terminal pro-sequence binds to an epitope comprised in the peptide sequence ranging from amino acid 323 to 334 of proSP-B. Detection is based on an electrochemiluminescence immunoassay (ECLIA), using a Tris(bipyridyl)-ruthenium(II) complex as label. For carrying out the assay, the biotinylated capture antibody (80 µl), the ruthenium-labeled detection antibody (80 µl), and sample or standard material (10 µl) are incubated in homogeneous phase for 9 min at 37°C. Concentrations in the stock solution were 1.7 µg/ml for the biotinylated capture antibody and 1.2 µg/ml for the ruthenylated detection antibody, respectively. After the first nine minutes 30 µl of Streptavidin-coated beads are added, and binding of the immune complexes formed to the microparticles takes place during a second 9-min incubation. After the second incubation, the reaction mixture is transferred into the measuring cell, where beads are captured to the electrode surface by a magnet. The measuring cell is washed to remove unbound label and filled with detection buffer containing Tris-propylamine. After applying voltage to the electrode, the emitted chemiluminescence light is detected by a photomultiplier. Results are determined via a 2-point calibration curve. The corresponding concentration for proSP-B is given in ng/ml.

The C-fragment proSP-B assay uses a first mouse monoclonal anti-proSP-B (C-terminus) antibody as a capture and a second mouse monoclonal anti proSP-B (C-terminus) antibody as a detection reagent. The assay principle is a sandwich format. The antibody to the first C-terminal pro-sequence binds to an epitope comprised in the peptide sequence ranging from amino acid 323 to 334 of proSP-B. The antibody to the second C-terminal pro-sequence binds to an epitope comprised in the peptide sequence ranging from amino acid 285 to 294 of proSP-B. Detection is based on an electrochemiluminescence immunoassay (ECLIA), using a Tris(bipyridyl)-ruthenium(II) complex as label. For carrying out the assay, the biotinylated capture antibody (MAB 1.7.41) (80 µl), the ruthenium-labeled detection antibody (MAB 1.3.9) (80 µl), and sample or standard material (10 µl) are incubated in homogeneous phase for 9 min at 37°C. Concentrations in the stock solution were 1.5 µg/ml for the biotinylated capture antibody and 1.0 µg/ml for the ruthenylated detection antibody, respectively. After the first nine minutes 30 µl of Streptavidin-coated beads are added, and binding of the immune complexes formed to the microparticles takes place during a second 9-min incubation. After the second incubation, the reaction mixture is transferred into the measuring cell, where beads are captured to the electrode surface by a magnet. The measuring cell is washed to remove unbound label and filled with detection buffer containing Tris-propylamine. After applying voltage to the electrode, the emitted chemiluminescence light is detected by a photomultiplier. Results are determined via a 2-point calibration curve. The corresponding concentration for C-terminal proSP-B is given in ng/ml.

NT-pro BNP and sensitive Troponin T were determined using commercially available ELECSYS tests.

NT-proBNP was determined with sandwich immunoassays using COBAS-analyzers from Roche/Hitachi. The assays comprise two monoclonal antibodies specific for the respective peptide. The first of these iv biotinylated and the second one in labelled with a Tris(2,2'-bibyridyl)ruthemium (II)-complex. In a first incubation step both antibodies are incubated with the sample. A sandwich complex comprising the peptide to be determined and the two different antibodies is formed. In a next incubation step streptavidin-coated beads are added to this complex. The beads bind the sandwich complexes. The reaction mixture is then aspirated into a measuring cell where the beads are magnetically captured on the surface of the electrode. The application of a voltage then induces a chemiluminescent emission from the ruthenium complex which is measured by a photomultiplier. The emitted amount of light is dependent on the amount of sandwich complexes on the electrode. NT-proBNP amounts between 2 pg/ml and 35,000 pg/ml can be measured.

Troponin T (hsTNT) was also tested using Roche analysers, the test follows the same test principles as described for NT-pro BNP. The high sensitivity Troponin T test used in this study has a sensitivity of 2 pg/ml and can be used on ELECSYS 2010 as well as on Cobas e411 an cobas e601 analysers.

### Example 2: proSP-B and C-terminal fragment of proSP-B are indicators for alveolar damage, alveolar hypoxia and/or left heart failure in pulmonary hypertension patients

A reference group consisted of 158 patients with coronary artery disease without lung disorder or pulmonary hypertension. They were asymptomatic with regard to heart failure, they had also no kidney disease as assessed by creatinine values within normal. Moreover they were clinically stable and specifically had no chest pain within 2 weeks prior to venal puncture.

A total of 104 patients with documented pulmonary hypertension were also included into the study. Pulmonary hypertension was considered to be present when the mean pulmonary artery pressure at rest exceeded 25 mmHg. Pre- from post- capillary hypertension was dissected according to the PCW (pulmonary capillary wedge pressure) at rest into pre-capillary (below 15 mmHg) and post-capillary (above 15 mmHg). Kidney function was normal in all patients, in addition, they were clinically stable judged on the absence of clinical worsening within the previous month.

The result of the reference population is shown in Table 1, below, patients were separated in two groups (above and below the median of NT-pro BNP (Median 116 pg/ml NT-pro BNP).

**Table 1: Biomarkers in reference population**

| | NT-pro BNP pg/ml | Troponin T pg/ml | proSP-B ng/ml | C-frag. proSP-B ng/ml |
|---|---|---|---|---|
| Group 1 | 54 | 1 | 25 | 50 |
| N=79 | (35 - 83) | (0-7) | (20 - 40) | (34 - 79 ) |
| Group 2 | 227 | 3 | 35 | 65 |
| N=79 | (153 - 292) | (0 - 9) | (26 - 55) | (45 - 105) |

The data obtained indicate that pro SP-B levels above 40 ng/ml and C-fragment pro SP-B above 79 ng/ml are abnormal in patients with normal cardiac function and pro-SP-B levels above 55 ng/ml and c fragment proSP-B above are abnormal in asymptomatic patients (without history of heart failure) but abnormal cardiac function (increased NT-pro BNP).

Patients with pulmonary arterial hypertension (PAH) were separated into tertiles based on the pro-SP-B values obtained. This is shown in Table 2, below.

**Table 2: Biomarkers in the tertile Cohorts for proSP-B in patients suffering from PAH**

| | Tertile 1 | Tertile 2 | Tertile 3 |
|---|---|---|---|
| proSP-B ng/ml | 48 (33/64) | 103 (88 / 123) | 245 (183/ 399) |
| C-frag proSP-B ng/ml | 91 (55/123) | 194 (157/246) | 619 (396/904) |
| NT-pro BNP pg/ml | 499 (230/2279) | 436 (186/1071) | 682 (245/2087) |
| Sens Troponin T pg/ml | 6 (3/19) | 13 (3/26) | 9 (3/17) |
| PCPW | 15 (11/24) | 14 (10/18 | 14 (10/20) |
| PA mean | 45 (37/53) | 41 (30/48) | 46 (37/60) |
| P 02 mmHg | 66 (59/76) | 64 (61/74) | 68 (58/75) |
| pCO2 mmHg | 35 (33/40) | 36 (33/41) | 34 (31/37) |

As can be easily derived from Table 2, proSP-B and C- fragment proSP-B is clearly increased in patients with PAH in the majority of patients when compared to patients/individuals without cardiac dysfunction or moderate (and asymptomatic cardiac dysfunction). ProSP-B did not correlate to NT-pro BNP or troponin T nor to indicators of pulmonary hypertension (PCPM, mean PA pressure) or peripheral markers of hypoxia (p0₂ and pC0₂) and thus provides independent information specifically to the state of alveolar type 2 cells.

Information on left ventricular systolic function from echocardiography was available from 92 patients, the results are summarised in Table 3, below:

**Table 3: Biomarkers in patients with normal and abnormal left ventricular systolic function according to echocardiography**

| | Systolic function | |
|---|---|---|
| | Normal | abnormal |
| proSP-B ng/ml | 107 (64/182) | 104 (71/ 136) |
| C-frag proSP-B ng/ml | 185 (120/383) | 236 (136/424) |
| NT-pro BNP pg/ml | 372 (218/ 984) | 1690 (194/6204) |
| Sens Troponin T pg/ml | 8 (3/17) | 19 (4/36) |

As can be seen form Table 3, patients with systolic dysfunction differed from those without detectable systolic dysfunction by echocardiography in terms of NT-pro BNP and sensitive Troponin T but not with respect to proSP-B and marginally with respect to C- fragment proSP-B.

A time course of the biomarkers was measured in order to determine the robustness of the proSP-B and C-terminal fragment proSP-B levels over time. Specifically, a total of 22 patients with clinically stable heart failure were included into the study, all patients were on standard heart failure therapy including beta blockers, ACE inhibitors and diuretics. Patients were seen at 2 weeks interval. During that period treatment was not changed. Moreover they have no change in symptoms und their weight did not differ of more than 2 kg bodyweight 4 weeks before study start and during the study. All patients had normal kidney function. Data are summarizes in the Table 4, below.

**Table 4: Time course measurements**

| | NT-pro BNP pg/ml | pro-SP-B ng/ml | c frag pro-SP-B ng/ml | TroponinT µg/ml |
|---|---|---|---|---|
| TP 1 | 367 (216-924) | 57 (33-80) | 119 (69-162) | 8 (4-15) |
| TP 2 | 362 (229-971) | 52 (31-75) | 111 (66-156) | 7 (3-12) |
| TP 3 | 374 (237-973) | 52 (29-75) | 109 (61-154) | 7 (4-12) |

As can be seen from the table pro-SP-B and c fragment pro SP-B are very stable and show little variation in this population indicating than minor changes in pro SP-B and c fragment pro SP-B respectively reflect changes in pulmonary-alveolar status.

In all groups studied, proSP-B provided similar or identical information when compared to C-terminal fragment proSP-B.

Conclusion:
ProSP-B and c fragment proSP-B provides information not captured by natriuretic peptides or troponin T and also not by other methods used in pulmonary arterial hypertension such as PCPW or mean PA.

Increased proSP-B may be attributable to left sided heart failure specifically in those patients who have evidence of left sided heart failure as documented by echocardiography or other methods. These patients require heart failure treatment and specifically diuretics.

It has also been documented that drugs resulting in vasodilatation (e.g. Prostacyclin) improve surfactant protein B production and alveolar function (possibly by reduction hypoxia; see Rose 1999, Am J. Respirat Crit Care Med 160: 846 - 851) and thus proSP-B can be used to direct and to improve pulmonary arterial hypertension therapy related to "alveolar health". Such drugs include prostaglandins, ET1 receptor blockers, nitric oxide and phosphodiesterase inhibitors.

In summary proSP-B offers a new and powerful new tool in the diagnosis of pulmonary arterial hypertension and offers new possibilities in directing its treatment.

## Claims

1. A method for diagnosing alveolar damage in a subject suffering from pulmonary hypertension, or not, said method comprising:
a) determining the amount of a surfactant protein-B peptide (SP-B peptide) in a sample of said subject; and
b) comparing said amount to a reference amount whereby it is diagnosed whether the said subject has alveolar damage, or not.

2. The method of claim 1, wherein said reference amount is derived from a subject or a group of subjects suffering from pulmonary hypertension known to have alveolar damage and wherein an essentially identical or increased amount of the SP-B peptide is indicative for a subject having alveolar damage, whereas a decreased amount of the SP-B peptide is indicative for a subject not having alveolar damage.

3. The method of claim 1, wherein said reference amount is derived from a subject or a group of subjects suffering from pulmonary hypertension known not to have alveolar damage and wherein an essentially identical or decreased amount of the SP-B peptide is indicative for a subject not having alveolar damage, whereas a increased amount of the SP-B peptide is indicative for a subject having alveolar damage.

4. The method of any one of claims 1 to 3, wherein said method further comprises determining the amount of a natriuretic and/or a cardiac troponin in said sample and comparing said amount(s) to a reference amount whereby it is diagnosed whether the said subject also exhibits left ventricular dysfunction, or not.

5. The method of any one of claims 1 to 4, wherein said method further comprises assessing an echocardiogram of said patient for left ventricular dysfunction.

6. The method of claim 4 or 5, wherein the diagnosis of alveolar damage and a left ventricular dysfunction is indicative for left heart failure.

7. The method of any one of claims 1 to 6, wherein said SP-B peptide is selected from the group consisting of: proSP-B, mature SP-B and C-terminal fragment of pro SP-B.

8. The method of any one of claims 1 to 7, wherein said subject does not exhibit impaired kidney function and/or is clinically stable with respect to the pulmonary hypertension.

9. The method of any one of claims 1 to 8, wherein said alveolar damage is damage of alveolar type 2 cells.

10. The method of any one of claims 1 to 9, wherein said method further comprises recommending a therapy for treating pulmonary hypertension associated with the said alveolar damage.

11. The method of claim 10, wherein said therapy for treating alveolar damage encompasses the administration of a drug selected from the group consisting of:
Prostaglandins, prostacyclins, such as epoprostenol, treproatinil, iloprost, or beraprost, ET1 receptor blockers, such as bosentan or ambrisentan, immunosuppressive agents, such as cyclophosphamide, nitric oxide and soluble guanylate cyclase stimulators, such as riociguat, Rho kinase inhibitors, such as fasudil, PDGF receptor inhibitors, such as imatinib, beta blockers, angiotensin receptor blockers, ACE inhibitors, and phosphodiesterase inhibitors, preferably, sildenafil.

12. The method of any one of claims 1 to 11, wherein said method further comprises recommending a monitoring intensiveness.

13. Use of a SP-B peptide or detection agents which specifically bind thereto in a sample of a subject exhibiting pulmonary hypertension for diagnosing alveolar damage.

14. A device adapted for carrying out the method of any one of claims 1 to 12 comprising
a) an analyzing unit comprising a detection agent which specifically binds to a SP-B peptide, said unit being adapted for determining the amount of the SP-B peptide in a sample of a subject exhibiting pulmonary hypertension; and
b) an evaluation unit for comparing the determined amount with a reference amount whereby it can be diagnosed whether the said subject has alveolar damage, said unit comprising a database with reference amount values and a computer-implemented algorithm carrying out the comparison.

15. A kit adapted for carrying out the method of any one of claims 1 to 12 comprising a detection agent for the SP-B peptide, reference standards as well as instructions for carrying out the said method.
